# EUROPÄISCHE PATENTSCHRIFT

(11) **EP 0 729 462 B1**
(45) Veröffentlichungstag und Bekanntmachung des Hinweises auf die Patenterteilung: **24.02.1999**
(21) Anmeldenummer: 95900684.2
(22) Anmeldetag: 09.11.1994
(51) Int. Cl.: C07D 307/32

(54) **VERFAHREN ZUR HERSTELLUNG VON GAMMA-BUTYROLACTON**
METHOD FOR PREPARING GAMMA-BUTYROLACTONE
PROCEDE DE PREPARATION DE GAMMA-BUTYROLACTONE

(30) Priorität: 18.11.1993 DE 4339269
(43) Veröffentlichungstag der Anmeldung: 04.09.1996
(73) Patentinhaber: BASF AKTIENGESELLSCHAFT, 67056 Ludwigshafen (DE)
(72) Erfinder: PINKOS, Rolf, D-67098 Bad Dürkheim (DE); FISCHER, Rolf, D-69121 Heidelberg (DE)
(86) Internationale Anmeldenummer: EP9403683
(87) Internationale Veröffentlichungsnummer: WO9514010

(56) Entgegenhaltungen:
- EP-A- 0 024 770
- CHEMICAL ABSTRACTS, vol. 108, no. 16, 18. April 1988, Columbus, Ohio, US; abstract no. 133838z, A. GHITA-DUMINICA ET AL. 'Catalytic manufacture of gamma-butyrolactone' Seite 109 ;Spalte 2 ;
- TETRAHEDRON LETTERS., Nr.39, September 1977, OXFORD GB Seiten 3483 - 3484 G. PIANCATELLI ET AL. 'Pyridinium Chlorochromate in the Organic Synthesis: A Convenient Oxidation of Enol-Ethers to Esters and Lactones' in der Anmeldung erwähnt

## Beschreibung

Die vorliegende Erfindung betrifft ein Verfahren zur Herstellung von γ-Butyrolacton.

Havel et al. (J. Org. Chem. 41, 513 (1976)) erzeugten γ-Butyrolacton durch die Oxidation von 2,3-Dihydrofuran mit Triplett-Sauerstoff. γ-Butyrolacton entsteht bei dieser Umsetzung nur in einer Ausbeute von 1,4 %. Piancatelli et al. (Tetrahedron Lett. 39, 3483 (1977)) oxidierten 2,3-Dihydrofuran mittels Pyridiniumchlorochromat. Das teure Oxidationsmittel Pyridinium-chlorochromat wird bei dieser Umsetzung in stöchiometrischen Mengen verbraucht, weshalb dieses Verfahren infolge mangelnder Wirtschaftlichkeit für eine technische Nutzung nicht in Frage kommt.

γ-Butyrolacton konnte ausgehend von 2,5-Dihydrofuran bislang nicht auf direktem Wege erhalten werden. Alper et al. (J. Mol. Cat. 72, 143 (1992)) beschrieben die Kobalt(II)chlorid-katalysierte Oxidation von 2,5-Dihydrofuran mit Sauerstoff zu 2-Buten-4-olid der Formel I 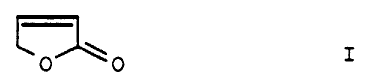 dessen C-C-Doppelbindung in einer nachfolgenden Umsetzung hydriert werden muß, bevor man zu γ-Butyrolacton gelangt. Somit ist auch dieses Verfahren unwirtschaftlich.

Gemäß EP-A 24 770 führt die Umsetzung von 2,5-Dihydrofuran in flüssiger Phase mit Wasser an einem Platin/Palladium auf Aluminiumoxid-Katalysator zu 4-Hydroxybutyraldehyd. γ-Butyrolacton wurde bei dieser Umsetzung nicht gefunden.

γ-Butyrolacton ist eine in großen Mengen benötigte Chemikalie, die beispielsweise als Lösungsmittel für Polyacrylnitril, Celluloseacetat, Polystyrol, Schellack und Harze dient und weiterhin als Ausgangsmaterial zur Herstellung von wirtschaftlich wichtigen Produkten, wie Pyrrolidon, N-Methylpyrrolidon und Polyvinylpyrrolidon verwendet wird.

Der vorliegenden Erfindung lag nun die Aufgabe zugrunde, ein Verfahren zu finden, das die Herstellung von γ-Butyrolacton, ausgehend von 2,5-Dihydrofuran oder 2,3-Dihydrofuran, auf wirtschaftliche Weise ermöglicht. Insbesondere sollte ein Weg gefunden werden, auf dem γ-Butyrolacton ausgehend von 2,5-Dihydrofuran direkt, in einer einzigen Stufe hergestellt werden kann.

Dementsprechend wurde ein Verfahren zur Herstellung von γ-Butyrolacton gefunden, das dadurch gekennzeichnet ist, daß man 2,5-Dihydrofuran oder 2,3-Dihydrofuran oder Gemische dieser beiden Dihydrofurane in der Gasphase in Gegenwart von Wasser und in An- oder Abwesenheit von zugesetztem Wasserstoff bei erhöhter Temperatur an einem Hydrierkatalysator umsetzt.

Zwar ist der chemische Reaktionsmechanismus für die dem erfindungsgemäßen Verfahren zugrundeliegende Umsetzung noch unbekannt, doch wird vermutet, daß diese Umsetzung nach dem folgenden Reaktionsschema ablaufen könnte. Danach wird vermutlich das 2,5-Dihydrofuran II zunächst am Hydrierkatalysator gemäß Gleichung (1) zu 2,3-Dihydrofuran III isomerisiert. 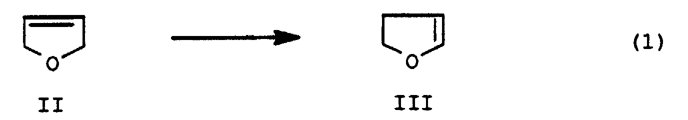 Anschließend könnte an das 2,3-Dihydrofuran gemäß Gleichung (2) Wasser addiert werden, 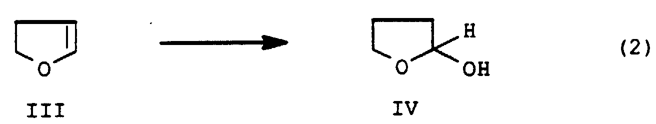 wobei sich 2-Hydroxytetrahydrofuran der Formel IV bildet, welches mit seinem offenkettigen Isomeren 4-Hydroxybutyraldehyd der Formel V 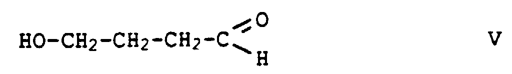 im Gleichgewicht steht. 2-Hydroxytetrahydrofuran IV wird dann vom Hydrierkatalysator vermutlich gemäß Gleichung (3) 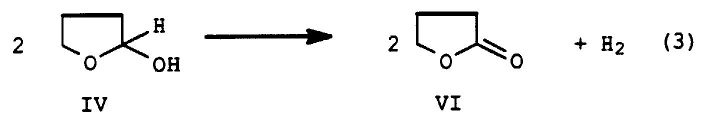 zu γ-Butyrolacton der Formel VI dehydriert. Der eingesetzte Hydrierkatalysator würde sonach als Dehydrierkatalysator wirken. Da die erfindungsgemäß verwendbaren Katalysatoren aber üblicherweise in der Technik als Hydrierkatalysatoren eingesetzt werden, werden diese Katalysatoren im Rahmen dieser Anmeldung trotz ihrer dehydrierenden Wirkung im erfindungsgemäßen Verfahren weiterhin als "Hydrierkatalysatoren" bezeichnet. Wie erwähnt, handelt es sich bei dem oben geschilderten Reaktionsmechanismus lediglich um den Versuch einer Erklärung für die im erfindungsgemäßen Verfahren am Katalysator ablaufenden chemischen Vorgänge, die im einzelnen nicht näher untersucht worden sind. Gestützt wird dieser Vorschlag für den Reaktionsmechanismus im wesentlichen durch die Beobachtungen, daß im erfindungsgemäßen Verfahren sowohl 2,5-Dihydrofuran als auch 2,3-Dihydrofuran eingesetzt werden können, das erfindungsgemäße Verfahren in Abwesenheit von zugesetztem Wasserstoff durchgeführt werden kann sowie durch die aufgefundenen Nebenprodukte.

Im erfindungsgemäßen Verfahren wird 2,5-Dihydrofuran oder 2,3-Dihydrofuran oder ein Gemisch dieser Verbindungen, vorzugsweise 2,5-Dihydrofuran alleine, in der Gasphase mit Wasser in einem Molverhältnis Dihydrofuran/Wasser von im allgemeinen 2:1 bis 1:100, vorzugsweise 1:1 bis 1:50 und besonders bevorzugt von 1:1 bis 1:10 in Gegenwart eines Hydrierkatalysators bei einem Druck von im allgemeinen 0,5 bis 50 bar, vorzugsweise 0,8 bis 40 bar und insbesondere von 1 bis 10 bar bei einer Temperatur von 100 bis 350°C, vorzugsweise von 130 bis 330°C und besonders bevorzugt von 170 bis 300°C zu γ-Butyrolacton umgesetzt.

Als Hydrierkatalysatoren können im erfindungsgemäßen Verfahren praktisch alle zur Hydrierung von Carbonylgruppen geeigneten Heterogenkatalysatoren verwendet werden, beispielsweise solche, wie sie in Houben-Weyl, Methoden der Organischen Chemie, Band IV, 1c, S. 16-26, Thieme-Verlag, Stuttgart, 1980, beschrieben werden. Die Hydrierkatalysatoren können im erfindungsgemäßen Verfahren in einem Festbett oder mobil, z.B. in einer Wirbelschicht, im Reaktor angeordnet werden.

Bevorzugt werden im erfindungsgemäßen Verfahren solche heterogene Hydrierkatalysatoren verwendet, die ein oder mehrere Elemente der Gruppen Ib, VIb, VIIb und VIIIb des Periodensystems der Elemente enthalten. Diese Katalysatoren können weiterhin zur Förderung ihrer katalytischen Aktivität und Selektivität zusätzlich ein oder mehrere Elemente aus den Gruppen Ia, IIa, IIIa, IVa und Va des Periodensystems der Elemente enthalten. Bevorzugte Katalysatoren sind insbesondere solche, die als katalytisch aktive Komponenten z.B. Kupfer, Chrom, Rhenium, Kobalt, Nickel, Rhodium, Ruthenium, Iridium, Palladium, Eisen oder Platin oder Gemische mehrerer dieser Elemente sowie gegebenenfalls als weitere, ihre katalytische Aktivität und Selektivität beeinflussende Komponenten z.B. Indium, Zinn oder Antimon enthalten. Besonders bevorzugt werden im erfindungsgemäßen Verfahren Hydrierkatalysatoren verwendet, die Rhenium und/oder Kupfer enthalten.

Als Heterogenkatalysatoren können im erfindungsgemäßen Verfahren sowohl sogenannte Fällungskatalysatoren als auch herkömmliche Trägerkatalysatoren eingesetzt werden, die durch Aufbringung der katalytisch aktiven Komponente auf ein Trägermaterial hergestellt worden sind.

Die Fällungskatalysatoren können hergestellt werden, indem man ihre katalytisch aktiven Komponenten aus deren Salzlösungen, insbesondere aus den Lösungen von deren Nitraten und/oder Acetaten durch Zugabe von Lösungen von Alkalimetall- und/oder Erdalkalimetallhydroxyd- und/oder Carbonatlösungen, z.B. als schwerlösliche Hydroxide, Oxidhydrate, basische Salze oder Carbonate ausfällt, den erhaltenen Niederschlag anschließend trocknet und diesen dann durch Calcinierung bei im allgemeinen 300 bis 700°C, insbesondere 400 bis 600°C in die entsprechenden Oxide, Mischoxide und/oder gemischtvalentigen Oxide umwandelt, welche durch eine Behandlung mit Wasserstoff oder Wasserstoff enthaltenden Gasen bei in der Regel 50 bis 700°C, insbesondere bei 100 bis 400°C zu den betreffenden Metallen und/oder oxidischen Verbindungen niederer Oxidationsstufe reduziert und in ihre eigentliche, katalytisch aktive Form überführt werden. Anstelle von Wasserstoff können zu diesem Zweck auch andere geeignete Reduktionsmittel, z.B. Hydrazin, verwendet werden, bevorzugt ist jedoch die Verwendung von Wasserstoff. Dabei wird in der Regel so lange reduziert, bis praktisch kein Wasserstoff mehr verbraucht wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Geeignete Trägermaterialien sind z.B. die Oxide des Aluminiums, des Titans, Zinkoxid, Zirkoniumdioxid, Siliciumdioxid, Tonerden, z.B. Montmorrillonite, Silikate, wie Magnesium- oder Aluminiumsilikate oder Zeolithe, wie ZSM-5- oder ZSM-10-Zeolithe. Es können auch Mischungen solcher Trägermaterialien verwendet werden. Gewünschtenfalls kann der getrocknete Niederschlag aus der Fällung vor der Calcinierung mit Formhilfsmitteln, wie Graphit, Talk oder Stearin, und/oder mit Porenbildnern wie Cellulose, Methylcellulose, Stärke, Wachs, Paraffin und/oder einem Polyalkylenglykol, versetzt und zu Katalysatorformkörpern wie Tabletten, Kugeln, Ringen oder Strängen verpreßt oder extrudiert werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren besonders gut, bei denen die katalytisch aktiv wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, beispielsweise mit Wasserstoff, Wasserstoff enthaltenden Gasen oder Hydrazin, vorzugsweise mit Wasserstoff enthaltenden Gasen, aufgebracht werden. Die Reduktion der auf dem Trägermaterial abgeschiedenen Metallverbindungen kann unter den gleichen Bedingungen erfolgen, wie sie zuvor bei den Fällungskatalysatoren geschildert wurden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. In diesem Falle können als Trägermaterialien auch Metalldrahtnetze oder Metallfolien dienen.

Der Gehalt der Trägerkatalysatoren an den katalytisch aktiven Metallen ist prinzipiell für das Gelingen der erfindungsgemäßen Verfahrens nicht kritisch. Es versteht sich für den Fachmann von selbst, daß höhere Gehalte dieser Trägerkatalysatoren an katalytisch aktiven Metallen zu höheren Raum-Zeit-Umsetzungen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-%, bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle, beispielsweise nach den Verfahren von DE-A 25 19 817, EP-A 147 219 und EP-A 285 420 auf den Träger aufgebracht werden. In den Katalysatoren gemäß den vorgenannten Schriften liegen die katalytisch aktiven Metalle als Legierungen vor, die durch thermische Behandlung und/oder Reduktion der z.B. durch Tränkung auf einem Träger abgeschiedenen Salze oder Komplexe der zuvor genannten Metalle, erzeugt werden.

Als Trägermaterialien können im allgemeinen die Oxide des Aluminiums, des Titans, Zinkoxid, Zirkoniumdioxid, Siliciumdioxid, Tonerden, z.B. Montmorillonite, Silikate, wie Magnesium- oder Aluminiumsilikate, Zeolithe, wie ZSM-5- oder ZSM-10-Zeolithe, sowie Aktivkohle verwendet werden. Bevorzugte Trägermaterialien sind Aluminiumoxide, Titandioxide, Siliziumdioxid, Zirkoniumdioxid oder Aktivkohle. Selbstverständlich können auch Mischungen verschiedener Trägermaterialien als Träger für die im erfindungsgemäßen Verfahren anwendbaren Hydrierkatalysatoren dienen.

Als für im erfindungsgemäßen Verfahren einsetzbare Hydrierkatalysatoren seien die folgenden beispielhaft genannt:
Platin auf Aktivkohle, Palladium auf Aktivkohle, Palladium auf Aluminiumoxid, Kobalt auf Aktivkohle, Kobalt auf Siliziumdioxid, Kobalt auf Aluminiumoxid, Eisen auf Aktivkohle, Mangan auf Aktivkohle, Rhenium auf Aktivkohle, Rhenium auf Siliziumdioxid, Rhenium/Zinn auf Aktivkohle, Rhenium/Palladium auf Aktivkohle, Kupfer auf Aktivkohle, Kupfer auf Siliziumdioxid, Kupfer auf Aluminiumoxid, Kupferchromit sowie die Katalysatoren gemäß DE-A 392 332, US-A 3 449 445, EP-A 44 444, EP-A 147 219, DE-A 39 04 083, DE-A 23 21 101, EP-A 415 202, DE-A 23 66 264 und EP-A 100 406.

Besonders bevorzugte Katalysatoren enthalten mindestens eines der Metalle Kupfer oder Rhenium. Kupferhaltige Fällungskatalysatoren können im allgemeinen 0,1 bis 90 Gew.-%, vorzugsweise 1 bis 80 Gew.-% und besonders bevorzugt 5 bis 50 Gew.-% Kupfer, berechnet als CuO und bezogen auf das Gesamtgewicht des Katalysators, enthalten. Kupferhaltige, durch Imprägnierung oder Beschichtung eines Trägermaterials hergestellte Trägerkatalysatoren können im allgemeinen 0,1 bis 30 Gew.-%, vorzugsweise 1 bis 30 Gew.-% und besonders bevorzugt 3 bis 25 Gew.-% Kupfer, berechnet als CuO und bezogen auf das Gesamtgewicht des Katalysators, enthalten. Rheniumhaltige Katalysatoren werden im erfindungsgemäßen Verfahren vorzugsweise in Form von Trägerkatalysatoren eingesetzt, die Rhenium, berechnet als Re und bezogen auf das Gesamtgewicht des Katalysators, in Mengen von 0,1 bis 25 Gew.-%, vorzugsweise von 1 bis 20 Gew.-% und besonders bevorzugt von 2 bis 10 Gew.-% enthalten.

Das erfindungsgemäße Verfahren wird vorteilhaft kontinuierlich ausgeübt. Dabei können beispielsweise Rohrreaktoren, in denen der Katalysator vorteilhaft in Form eines Festbetts angeordnet ist, oder Wirbelschichtreaktoren, in denen der Katalysator durch den Gasstrom bewegt wird, eingesetzt werden.

Die Edukte 2,5-Dihydrofuran und/oder 2,3-Dihydrofuran und Wasser können z.B. in einem Verdampfer in die Gasphase überführt werden, bevor sie über den Katalysator geleitet werden. Vorteilhaft werden die Edukte in einem Trägergasstrom verdampft, wobei als Trägergase beispielsweise Edelgase, Stickstoff, C₁- bis C₄-Kohlenwasserstoffe, vorzugsweise Methan und bevorzugt Wasserstoff eingesetzt werden können. Der Trägergasstrom wird dabei so dosiert, daß die Edukte, Produkte und gegebenenfalls entstehende Nebenprodukte, z.B. 1,4-Butandiol, Tetrahydrofuran und Butanol, im Reaktor gasförmig bleiben.

Der zur Verdampfung der Edukte dienende Trägergasstrom kann vorteilhaft auch im Kreis geführt werden, d.h. nach Abscheidung der in ihm nach Verlassen der Katalysatorschüttung enthaltenen Produkte, beispielsweise in einem Gas-Flüssigkeits-Abscheider oder Kühler, erneut zur Verdampfung der Edukte als Trägergasstrom wiederverwendet werden. Wird keine Kreisgasfahrweise angewandt oder ein anderes Trägergas als Wasserstoff verwendet, so genügt der bei der Umsetzung gemäß Gleichung (3) am Katalysator freigesetzte Wasserstoff, um den Hydrierkatalysator aktiv zu halten.

Im allgemeinen werden die Katalysatoren im erfindungsgemäßen Verfahren mit 0,05 bis 5 kg Dihydrofuran/1 Katalysator·h⁻¹, vorzugsweise mit 0,1 bis 3 kg Dihydrofuran/1 Katalysator·h⁻¹ belastet.

Der gasförmige Reaktoraustrag kann nach Abkühlung und Kondensation der Produkte auf an sich herkömmliche Weise destillativ aufgearbeitet werden, beispielsweise durch fraktionierte Destillation. Es ist auch möglich, den gasförmigen Reaktionsaustrag direkt in eine Destillationskolonne zu leiten. Bei der Destillation können außer dem Verfahrensprodukt γ-Butyrolacton auch gegebenenfalls im Reaktoraustrag enthaltene Nebenprodukte, wie 1,4-Butandiol, Tetrahydrofuran und/oder n-Butanol, als Wertprodukte gewonnen werden. Gegebenenfalls im Reaktoraustrag enthaltenes 2,5- und/oder 2,3-Dihydrofuran und/oder Wasser kann bei der Destillation vom Produkt abgetrennt und wieder, nach Verdampfung, in den Reaktor zurückgeführt werden.

Das erfindungsgemäße Verfahren ermöglicht somit ausgehend von 2,5-Dihydrofuran und/oder 2,3-Dihydrofuran die wirtschaftliche Herstellung von γ-Butyrolacton. Besonders vorteilhaft ist dabei, daß γ-Butyrolacton in einer Stufe aus 2,5-Dihydrofuran erzeugt werden kann.

Das erfindungsgemäß verwendete Ausgangsmaterial 2,5-Dihydrofuran kann durch Isomerisierung von Vinyloxiran, beispielsweise nach den Verfahren gemäß US-A 5 034 545 und US-A 5 082 956, hergestellt werden. 2,3-Dihydrofuran kann aus 2,5-Dihydrofuran durch dessen basenkatalysierte oder photochemisch katalysierte Isomerisierung, beispielsweise nach den Verfahren von Paul et al. (Bull. Soc. Chim. France 668 (1950)) oder Hubert et al. (J. Chem. Soc. Perkin II 366 (1972)), gewonnen werden.

### Beispiele

Die in den folgenden Beispielen angegebenen Selektivitäten (Ausbeute/Umsatz·100) wurden gaschromatographisch unter Verwendung eines inneren Standards bestimmt.

### Beispiel 1

In einen 160 ml Gasphasenreaktor mit äußerer Heizung wurden 102 g eines Kupfer auf Siliziumdioxid-Trägerkatalysators (Kupfergehalt: 22 Gew.-%, berechnet als CuO und bezogen auf das Gesamtgewicht des Katalysators; hergestellt durch Tränkung des Trägers mit einer ammoniakalischen Kupfercarbonat-Lösung, Trocknung des imprägnierten Trägers bei 120°C und Calcinierung bei 500°C) in Form von Splitt gefüllt und im Wasserstoffstrom bei einer Temperatur von anfänglich 150°C und einer Endtemperatur von 250°C reduziert. Danach wurden über einen Vorverdampfer 33 ml/h Wasser und 16 ml/h 2,5-Dihydrofuran in einem Wasserstoffstrom von 18 l/h bei 200°C/1013 mbar kontinuierlich verdampft und in den 210°C heißen Reaktor geleitet. Der Reaktoraustrag wurde in einer gekühlten Vorlage aufgefangen und analysiert. Bei einem Umsatz von 58 % wurde γ-Butyrolacton mit einer Selektivität von 83 % erhalten (2,3-Dihydrofuran: 0,4 %; Furan: 4 %; Tetrahydrofuran: 9 %; n-Butanol: 3 %; Rest: geringe Mengen verschiedener Leichtsieder, wie Propanol, die nicht weiter bilanziert wurden).

### Beispiel 2

Analog Beispiel 1 wurden 15 ml/h 2,5-Dihydrofuran und 32 ml/h Wasser bei 220°C über 149 g eines Kupfer und Magnesium auf Siliziumdioxid enthaltenden Katalysators (Zusammensetzung: Kupfer, berechnet als CuO: 43,0 Gew.-%; Magnesium, berechnet als MgO: 18,0 Gew.-%; Silikat, berechnet als SiO₂: 35,0 Gew.-%; Barium, berechnet als BaO: 1 Gew.-%; Chrom, berechnet als Cr₂O₃: 0,6 Gew.-%; Zink, berechnet als ZnO: 0,4 Gew.-%; Natrium, berechnet als Na₂O: 0,2 Gew.-%; der Rest ist vorwiegend Carbonat; alle Angaben bezogen auf das Gesamtgewicht des Katalysators; hergestellt durch eine gemeinsame Fällung aus einer Lösung der Metallsalze und Natriumsilikat (Wasserglas) mit Natriumcarbonat, Trocknung des erhaltenen Niederschlags, Extrudierung zu Strängen mit Talk als Verformungshilfsmittel und Calcinierung bei 500°C, Reduktion analog Beispiel 1) geleitet. Bei einem Umsatz von 97 % wurde γ-Butyrolacton mit einer Selektivität von 85 % erhalten (2,3-Dihydrofuran: 1,4 %; Furan: 3 %; Tetrahydrofuran: 9 %; n-Butanol: 1 %; Rest: geringe Mengen verschiedener, nicht bilanzierter Leichtsieder).

### Beispiel 3

Analog Beispiel 1 wurden 19 ml/h 2,5-Dihydrofuran und 11 ml/h Wasser über 142 g des Katalysators aus Beispiel 2 geleitet. Bei einem Umsatz von 99,7 % wurde γ-Butyrolacton mit einer Selektivität von 89 % erhalten (2,3-Dihydrofuran: 0,13 %; Furan: 3,9 %; Tetrahydrofuran: 5 %; n-Butanol: 1,7 %; Rest: geringe Mengen verschiedener, nicht bilansierter Leichtsieder).

### Beispiel 4

Analog Beispiel 1 wurden 8 ml/h 2,5-Dihydrofuran und 15 ml/h Wasser bei 210°C über 85 g eines Kupfer auf Aktivkohle-Katalysators (Kupfergehalt: 10 Gew.-%, berechnet als CuO und bezogen auf das Gesamtgewicht des Katalysators; hergestellt durch Imprägnierung von 4 mm-Aktivkohlesträngen mit ammoniakalischer Kupfercarbonatlösung; Trocknung bei 120°C und Reduktion analog Beispiel 1) geleitet. Bei einem Umsatz von 98 % wurde γ-Butyrolacton mit einer Selektivität von 83 % gebildet (2,3-Dihydrofuran: 2 %; Furan: 4 %; Tetrahydrofuran: 4 %; n-Butanol: 1 %; 4-Hydroxybutyraldehyd: 5 %; 1,4-Butandiol: 0,2 %; Rest: geringe Mengen verschiedener, nicht bilanzierter Leichtsieder).

### Beispiel 5

Analog Beispiel 1 wurden 10 ml/h 2,5-Dihydrofuran und 9 ml/h Wasser über 73 g eines Rhenium auf Aktivkohle-Katalysators (Rheniumgehalt: 6 Gew.-%, berechnet als Re; hergestellt durch Imprägnierung von 4 mm-Aktivkohlesträngen mit einer wäßrigen Dirheniumheptoxid (Re₂O₇)-Lösung; Trocknung bei 120°C; Reduktion analog Beispiel 1) geleitet. Bei einem Umsatz von 99 % wurde γ-Butyrolacton mit einer Selektivität von 91 % gebildet (2,3-Dihydrofuran: 0,5 %; Furan: 1 %; Tetrahydrofuran: 5 %; n-Butanol: 1 %; Rest: geringe Mengen verschiedener, nicht bilanzierter Leichtsieder).

### Beispiel 6

Analog Beispiel 5 wurden über den Rhenium auf Aktivkohle-Katalysator 14 ml/h 2,5-Dihydrofuran und 24 ml/h Wasser bei 220°C geleitet. Bei vollständigem Umsatz wurde γ-Butyrolacton mit einer Selektivität von 87 % gebildet (Furan: 2,5 %; Tetrahydrofuran: 9 %; n-Butanol: 1 %; Rest: verschiedene, nicht bilanzierte Leichtsieder).

### Beispiel 7

Analog Beispiel 1 wurden 16 ml/h 2,5-Dihydrofuran und 30 ml/h Wasser bei 230°C über 253 g des käuflichen Kupferchromit-Katalysators der Fa. Südchemie, München, mit der Bezeichnung G 22 (Zusammensetzung gemäß Verkaufsprospekt: 37 % Cu; 46 % Cr₂O₃; 13 % BaO; Reduktion analog Beispiel 1) geleitet. Bei einem Umsatz von 71 % wurde γ-Butyrolacton mit einer Selektivität von 67 % gebildet (2,3-Dihydrofuran: 8 %; Furan: 3 %; Tetrahydrofuran: 2 %; n-Butanol: 1 %; 4-Hydroxybutyraldehyd: 0,5 %; Rest: verschiedene, nicht bilanzierte Leichtsieder).

### Beispiel 8

Analog Beispiel 1 wurden 10 ml/h 2,3-Dihydrofuran und 8 ml/h Wasser bei 220°C in einem Trägergasstrom von 13 l/h Wasserstoff über 47 g des Rhenium auf Aktivkohle-Katalysators gemäß Beispiel 5 geleitet (Reaktorvolumen: 100 ml). Bei einem Umsatz von 99,5 % wurde γ-Butyrolacton mit einer Selektivität von 98 % gebildet (Tetrahydrofuran: 0,3 %; 4-Hydroxybutyraldehyd: 0,5 %; Rest: geringe Mengen verschiedener, nicht bilanzierter Leichtsieder).

### Beispiel 9

Analog Beispiel 8 wurden 10 ml/h 2,3-Dihydrofuran und 10 ml/h Wasser bei 220°C in einem Trägergasstrom von 13 l/h Wasserstoff über 45 g eines Rhenium-Palladium auf Aktivkohle-Katalysators (Rheniumgehalt: 3 Gew.-%, berechnet als Re; Palladiumgehalt: 3 Gew.-%, berechnet als Pd; jeweils bezogen auf das Gesamtgewicht des Katalysators; hergestellt durch Tränken von 4 mm-Aktivkohlesträngen mit einer Lösung von PdCl₂ in wäßriger Salzsäure, Trocknung und erneuter Tränkung des Trägers mit einer wäßrigen Dirheniumheptoxid (Re₂O₇)-Lösung; Trocknung bei 120°C und Reduktion gemäß Beispiel 1) geleitet. Bei einem Umsatz von 99,5 % wurde γ-Butyrolacton mit einer Selektivität von 1 % gebildet.

Nach Umstellung des Trägerstroms von Wasserstoff auf 12 l/h Stickstoff wurde bei vollständigem Umsatz γ-Butyrolacton mit einer Selektivität von 15 % erhalten.

## Patentansprüche

1. Verfahren zur Herstellung von γ-Butyrolacton, dadurch gekennzeichnet, daß man 2,5-Dihydrofuran oder 2,3-Dihydrofuran oder Gemische dieser beiden Dihydrofurane in der Gasphase in der Gegenwart von Wasser und in An- oder Abwesenheit von zugesetztem Wasserstoff bei erhöhter Temperatur an einem Hydrierkatalysator umsetzt.

2. Verfahren nach Anspruch 1, dadurch gekennzeichnet, daß man die Umsetzung in Gegenwart von zugesetztem Wasserstoff durchführt.

3. Verfahren nach den Ansprüchen 1 und 2, dadurch gekennzeichnet, daß man die Umsetzung in Gegenwart von zugesetztem Wasserstoff durchführt und wobei der Wasserstoff als Trägergas dient.

4. Verfahren nach den Ansprüchen 1 bis 3, dadurch gekennzeichnet, daß man die Umsetzung bei einer Temperatur von 100 bis 350°C und bei einem Druck von 0,5 bis 50 bar durchführt.

5. Verfahren nach den Ansprüchen 1 bis 4, dadurch gekennzeichnet, daß man einen Hydrierkatalysator verwendet, der mindestens ein Element aus den Gruppen Ib, VIb, VIIb und/oder VIIIb des Periodensystems der Elemente enthält.

6. Verfahren nach den Ansprüchen 1 bis 5, dadurch gekennzeichnet, daß man einen Hydrierkatalysator verwendet, der außer mindestens einem Element aus den Gruppen Ib, VIb, VIIb und/ oder VIIIb noch mindestens ein Element aus den Gruppen IIb, Ia, IIa, IIIa, Va und/oder VIa des Periodensystems der Elemente enthält.

7. Verfahren nach den Ansprüchen 1 bis 6, dadurch gekennzeichnet, daß man einen Hydrierkatalysator verwendet, der Rhenium enthält.

8. Verfahren nach den Ansprüchen 1 bis 6, dadurch gekennzeichnet, daß man einen Hydrierkatalysator verwendet, der Kupfer enthält.

9. Verfahren nach den Ansprüchen 1 bis 8, dadurch gekennzeichnet, daß man als Hydrierkatalysator einen Trägerkatalysator verwendet.

10. Verfahren nach den Ansprüchen 1 bis 9, dadurch gekennzeichnet, daß man den Hydrierkatalysator vor Beginn der Umsetzung mit Wasserstoff oder Wasserstoff enthaltenden Gasen reduziert.

## Claims

1. A process for preparing γ-butyrolactone, which comprises reacting 2,5-dihydrofuran or 2,3-dihydrofuran or mixtures of these two dihydrofurans in the gas phase in the presence of water and in the presence or absence of added hydrogen at elevated temperatures over a hydrogenation catalyst.

2. A process as claimed in claim 1, wherein the reaction is carried out in the presence of added hydrogen.

3. A process as claimed in claim 1 or 2, wherein the reaction is carried out in the presence of added hydrogen and the hydrogen serves as carrier gas.

4. A process as claimed in any of claims 1 to 3, wherein the reaction is carried out at from 100 to 350°C and at a pressure of from 0.5 to 50 bar.

5. A process as claimed in any of claims 1 to 4, wherein the hydrogenation catalyst used comprises at least one element selected from groups Ib, VIb, VIIb and/ or VIIIb of the Period Table of the Elements.

6. A process as claimed in any of Claims 1 to 5, wherein the hydrogenation catalyst used comprises not only at least one element selected from groups Ib, VIb, VIIb and/or VIIIb but also at least one element selected from groups IIb, Ia, IIa, IIIa, Va and/or VIa of the Periodic Table of the Elements.

7. A process as claimed in any of claims 1 to 6, wherein the hydrogenation catalyst used comprises rhenium.

8. A process as claimed in any of claims 1 to 6, wherein the hydrogenation catalyst used comprises copper.

9. A process as claimed in any of claims 1 to 8, wherein the hydrogenation catalyst used is a supported catalyst.

10. A process as claimed in any of claims 1 to 9, wherein the hydrogenation catalyst is reduced by means of hydrogen or hydrogen-containing gases before commencement of the reaction.

## Revendications

1. Procédé de préparation de γ-butyrolactone, caractérisé en ce que l'on fait réagir à hautes températures sur un catalyseur d'hydrogénation, du 2,5-dihydrofurane ou du 2,3-dihydrofurane ou des mélanges de ces deux dihydrofuranes, en phase gazeuse en présence d'eau et en présence ou en l'absence d'hydrogène ajouté.

2. Procédé selon la revendication 1, caractérisé en ce que l'on mène la réaction en présence d'hydrogène ajouté.

3. Procédé selon les revendications 1 ou 2, caractérisé en ce que l'on mène la réaction en présence d'hydrogène ajouté et où l'hydrogène sert de gaz porteur.

4. Procédé selon l'une quelconque des revendications 1 à 3, caractérisé en ce que l'on mène la réaction à une température de 100-350°C et sous une pression de 0,5-50 bar.

5. Procédé selon l'une quelconque des revendications 1 à 4, caractérisé en ce que l'on utilise un catalyseur d'hydrogénation contenant au moins un élément choisi dans les groupes Ib, VIb, VIIb et/ou VIII de la classification périodique des éléments.

6. Procédé selon l'une quelconque des revendications 1 à 5, caractérisé en ce que l'on utilise un catalyseur d'hydrogénation contenant, en plus d'au moins un élément choisi dans les groupes Ib, VIb, VIIb et/ou VIII de la classification périodique des éléments, au moins un autre élément choisi dans les groupes IIb, Ia, IIa, IIIa, Va et/ou VIa de la classification périodique des éléments.

7. Procédé selon l'une quelconque des revendications 1 à 6, caractérisé en ce que l'on utilise un catalyseur d'hydrogénation contenant du rhénium.

8. Procédé selon l'une quelconque des revendications 1 à 6, caractérisé en ce que l'on utilise un catalyseur d'hydrogénation contenant du cuivre.

9. Procédé selon l'une quelconque des revendications 1 à 8, caractérisé en ce que l'on utilise un catalyseur supporté en tant que catalyseur d'hydrogénation.

10. Procédé selon l'une quelconque des revendications 1 à 9, caractérisé en ce que l'on réduit le catalyseur d'hydrogénation avant le début de la réaction, à l'aide d'hydrogène ou de gaz contenant de l'hydrogène.
